# EUROPEAN PATENT APPLICATION

(11) **EP 3 936 146 A1**
(43) Date of publication of application: **12.01.2022**
(21) Application number: 20757210.8
(22) Date of filing: 16.03.2020
(51) Int. Cl.: A61K 38/18, A61K 9/00, A61P 17/02

(54) **USE OF EPIDERMAL GROWTH FACTOR IN DIABETIC FOOT ULCER TREATMENT**

(30) Priority: 18.03.2019 CU 20190022
(71) Applicant: Centro De Ingeniería Genética Y Biotecnología, 11600 La Habana (CU)
(72) Inventor: DEL RIO MARTIN, Amaurys, La Habana 11 100 (CU); SAURI CHAVEZ, Jose, Esteban, La Habana 12 700 (CU); UBIETA GÓMEZ, Raimundo, La Habana 11 600 (CU); GONZALEZ BLANCO, Sonia, La Habana 11 600 (CU); BERLANGA ACOSTA, Jorge, Amador, La Habana 17 100 (CU); MUZIO GONZÁLEZ, Verena, Lucila, La Habana 11 600 (CU); PIMENTEL VÁZQUEZ, Eulogio, La Habana 11 600 (CU)
(74) Representative: V.O.
(86) International application number: PCT/CU2020/050003
(87) International publication number: WO 2020/187341

(57) **Abstract**

The present invention reveals the use of epidermal growth factor (EGF) for the manufacture of an injectable medicament for the treatment of a diabetic foot ulcer (DFU), in a patient without criteria of amputation of the affected limb at the time of the clinical evaluation, wherein the medicament comprising EGF is administered by intralesional infiltration in said ulcer once a week. Moreover, it provides a pharmaceutical composition comprising EGF, for the treatment of a DFU in a patient, where the composition is administered once a week. The invention comprises a method for the treatment of a DFU in a patient, without criteria of amputation of the affected limb at the time of the clinical evaluation, comprising administering EGF to the patient, by intralesional infiltration in the ulcer base once a week.

## Description

### Technical field

The present invention is related to the fields of human medicine and pharmaceutical industry. In particular, it refers to the use of epidermal growth factor (EGF) for manufacturing of a medicament for the treatment of diabetic foot ulcer (DFU). This medicament is administered by intralesional infiltration into the wound base.

### Previous art

Diabetes mellitus constitutes a major problem for public health services and it is one of the most common chronic non-communicable diseases. The International Diabetes Federation states that this disease has a global prevalence in adults of 8.3%. According to statistical data from the World Health Organization, by 2045 a diabetic population exceeding 629 million people is forecast (International Diabetes Federation. The global burden. In: International Diabetes Federation. IDF Diabetes Atlas. Geneva: IDF; 2017 p. 9-50*).* Currently, diabetes continues to show an accelerated growth, mainly due to bad dietary habits and the systematic inactivity, which has placed it as a non-communicable disease with global pandemic outreach. The therapeutic education of patients with diabetes mellitus is the basis of their treatment. Ignorance in self-care measures and lack of periodic outpatient follow-up favor complications (Al-Wahbi AM. Vasc Health Risk Manag. 2010; 6: 923-34*;* Dorresteijn J, Valk JD. Diabetes Metab Res Rev 2012; 28 (Suppl 1): 101-6) such as DFU. Although prevention programs have been developed, and health system services are organized, amputations are still an important problem pending to be solved (García Garcia Y, et al. Cuban Journal of Angiology and Vascular Surgery 2016; 17 (1) 36-43).

The concept of diabetic foot is difficult to define precisely (Apelqvist J, et al.; International Working Group on the Diabetic Foot Editorial Board. Diabetes Metab Res Rev 2008: 24 (Suppl 1): S181-7)*.* In the *2007 International Diabetic Foot Consensus* document, the diabetic foot is defined as "ulceration, infection or destruction of deep tissues associated with neuropathy and / or peripheral arterial disease in the lower extremities of people with diabetes". It is considered "foot in risk" when some factors that transform it into a foot vulnerable to ulceration, such as neuropathy, ischemia, deformity, calluses and edema are present (García Herrera AL, Febles Sanabria R, Moliner Cartaya M. Revista Cuban of Angiology and Vascular Surgery 2016; 17 (1): 13-24).

For the treatment of DFU, in the state of the art there are different types of treatment, approved by international consensus and published in high-impact journals, such as the *National Institute for Health and Care Excellence of the United Kingdom* (NICE), among others. Within these can be stated the discharges of pressure sites, vacuum therapy, hyperbaric oxygenation, dressings with varied properties (which are applied according to the type of lesion) and broad-spectrum antimicrobials, alone or in combination. There are also the different types of debridement, whether mechanical, biological or chemical. In addition, skin substitutes are applied to these patients (https://nice.org.uk/guidance/ng19, publication date: August 2015). Nonetheless, none of these treatments has been able to reduce the high percentage of amputations that are performed annually due to DFU. Healing of the ulcer is the most significant event for the patient. However, granulation, as an intermediate event that correlates with the lesion closure, acquires an important connotation, since it reduces the risk of infection of the ulcer and the risk of affected limb amputation, in lesions that do not respond to usual or standard treatments.

Since the beginning of the 90's, the administration of growth factors was evaluated in the treatment of chronic wounds. One of these growth factors is EGF, a low molecular weight protein, known since the 50's of the past century (Hardwicke J et al. Surgeon 2008; 6: 172-177). In trials of administration in chronic wounds, venous ulcers and DFU, this growth factor has been repeatedly applied by topical route. The microenvironment in the chronic diabetic wounds is hostile for the stability of local growth factors, their chemical integrity, their bioavailability and for their physiological role as main drivers of the healing process. Within this environment, the constant expression of their receptors and their signaling capacity are impaired (Berlanga-Acosta J, et al. Int Wound J 2013; 10: 232-236*;* Tsourdi E, et al. Biomed Res Int 2013: 385641).

It is known that the improvement of wound healing by EGF is promoted under a system of prolonged, sustained and slow release, which keeps its receptor constantly expressed. According to the state of the art, a constant exposure of the EGF to its receptor is required. This means that the wound healing properties of EGF could be expressed if the receptors are exposed, and constantly occupied, for at least 8 to 12 hours (Knauer DJ et al. J Biol Chem 1984, 259: 5623-5631*;* Buckley A et al. Proc Natl Acad Sci USA 1985, 82: 7340-7344).

On the other hand, it has been shown that prolonged local bioavailability, and timely stimulation of the receptor, is required for a significant impact mediated by the EGF in wound closure (Kasayama S, Ohba Y, Oka T Proc Natl Acad Sci USA 1989; 86: 7644-7648*;* Berlanga-Acosta J Int Wound J 2011; 8: 612-620*;* Cama VF et al. J Diabetic Complications Med 2016; 1: 111).

A lyophilized product for intralesional use, whose active principle is recombinant human Epidermal Growth Factor (rhEGF), was approved as a medicament for the treatment of DFU. Intralesional application of the drug provides the recombinant protein to the ulcer base (replacement therapy), since the microenvironment cells of the lesion cannot synthesize the endogenous protein, or do so in low proportions. This lack interferes with the ulcer healing, which leads to chronicity, and consequently to the surge of complications, such as local infection and amputation of the affected limb. The aforementioned medicament reduces amputation rates by 70% or more, with the highest reduction percentages in those less complicated ulcers. The intralesional administration of the rhEGF shows a positive benefit profile. Thousands of DFU patients have been treated with injectable rhEGF, administered by this route.

The infiltration of rhEGF at the edges and bottom of the DFU is carried out in an schedule of treatment of three times per week, and 75 µg of factor per dose of administered medicament. Starting from said administration, there is an awakening of the senescent cells, especially fibroblasts and keratinocytes, and it starts a change from a chronic pro-inflammatory environment, in the ulcer area, towards a physiological repair environment. It guarantees a proper granulation or useful vital tissue, and subsequently the lesion closure. The granulation process allows a correlation with the lesion closure of more than 80%. Despite the high correlation between granulation and closure, when DFU patients with moderate ischemia of the affected limb are incorporated into treatment, these percentages may diverge.

The administration schedule of injectable rhEGF including three times a week, and 75 µg per administered dose, has the disadvantage of frequent occurrence of adverse events and the high cost of the treatment, as well as discomfort to the patient, which could cause his withdrawal from the therapy.

Therefore, it is necessary to identify new administration schemes for injectable rhEGF, which allow the correct granulation and effective lesion closure in a patient suffering from diabetic foot, while increasing the well-being of the individual and their adherence to treatment.

### Detailed description of the invention

The present invention solves the problem mentioned above, by revealing the use of epidermal growth factor (EGF) for the manufacture of an injectable medicament for the treatment of a DFU, in a patient without criteria for amputation of the affected limb at the time of evaluation, and where the medicament comprising EGF is administered by intralesional infiltration into the ulcer base once a week. In an embodiment of the invention the amount of EGF per dose of the medicament administered to the patient goes from 2 µg to 80 µg.

To carry out the invention, the evaluation of the DFU in the patient can be performed at the beginning, when the initial diagnosis is made, or during the course of a treatment to prevent the amputation of the limb affected by the ulcer. When it is considered that there is no amputation criterion, the medicament containing the EGF can be administered by intralesional infiltration into the ulcer base once a week. In the invention, the patient is treated with the conventional therapy, established by medical authorities, while receiving intralesional infiltration of EGF in the base of the DFU once a week.

In an embodiment of the invention, the patient has previously received a treatment to prevent amputation of the limb affected by the ulcer. This previous treatment consists in the administration, three times a week, of EGF by intralesional route. Once the DFU is evaluated, and it is verified that there is no amputation criterion of the affected limb, the treatment is changed to intralesional infiltration of EGF in the ulcer base once a week.

To date, the EGF has been administered intralesionally to thousands of DFU patients, in a treatment that includes three applications of the drug per week. However, there is no evidence of efficacy of an administration regimen where the EGF is administered once a week, either during part of the period or during the entire course of treatment. The result of effective closure of the DFU with this new therapy protocol, of lower frequency of application of the EGF, cannot be anticipated starting from what it is described in the previous literature, since little is known about the healing mechanism in diabetic patients, well distant from what takes place in non-diabetic individuals.

On the other hand, as stated above, it is known that the improvement of wound healing by EGF is promoted under a system of prolonged, sustained and slow release, and for that a prolonged local bioavailability and timely stimulation of the receptor is required. For all this previous knowledge, reported in the literature within this technical field, it was not expected that after a decrease in the frequency of application of EGF to DFU the results were similar to those achieved with a more frequent application, even in patients without criteria for amputation of the affected limb, as it is the case of this invention. Rather, it would be expected that a treatment as spaced as once a week would induce a decrease in the expression of the EGF receptor and, therefore, a negative response to therapy with this growth factor. For this reason, the first trials to attempt to reduce the frequency of application were performed with a tripled dose of EGF (225 µg), which intended to maintain a sufficient amount of growth factor for a longer time in the ulcer. Unexpectedly, it was found that the residual amount of rhEGF in the ulcer was the same as with usual doses, and that the same therapeutic effect was maintained. It was even more striking that the same satisfactory results were obtained with the usual doses and with only one weekly application in this type of ulcers.

In the context of the invention, epidermal growth factor (EGF) refers to any of the variants of EGF molecules that maintain their biological activity; for example molecules truncated by the C-terminus (Calnan et al., Gut 2000, 47: 622-627); or truncated by the N-terminal end (Shin et al., Peptides 1995, 16: 205-210). Human EGF can be obtained by recombinant deoxyribonucleic acid technology (rhEGF) in hosts well known to those skilled in the art. Without limitating the scope of the invention, among these hosts are yeasts, for example, of the genus *Saccharomyces* or *Pichia*; or bacteria like *Escherichia coli.* The polypeptide can also be obtained by chemical synthesis.

In the invention, it is considered that the limb affected by DFU does not comply with amputation criterion when the tissues have the following conditions: Slight or discreetly moderate degree of ischemia; do not show signs of local infection, given by secretion, unpleasant smell, bone involvement, such as osteomyelitis; do not show inflammatory signs such as edema, heat, flushing, pain and loss of functionality in the area of the ulcer that have an impact on the general condition of the patient, that is, that the patient does not have signs and symptoms of generalized sepsis; do not have extensive muscle necrosis; that have a bottom with good tonicity, with easy bleeding during the healing process; that have sensitivity as best conserved as possible, that is, that the patient show discomfort during the healing process.

The term 'DFU evaluation' is well known by the skilled in the field, and it appears in the therapeutic guidelines established by medical colleges and authorities. On the other hand, for the purposes of the invention, the term infiltration of EGF refers to the injection at various points of the DFU base.

In another aspect, the invention provides a pharmaceutical composition for the treatment of DFU in a patient, without amputation criteria of the affected limb at the time of the evaluation, comprising rhEGF and pharmaceutically acceptable excipients. Said composition is administered once a week. In an embodiment of the invention, the composition comprises an amount of EGF between 2 and 80 µg per dose administered to the patient. Without limitating the scope, for the pharmaceutical composition, the EGF is formulated with excipients and vehicles like stabilizers, buffers, and compounds that allow lyophilization without affecting the biological activity of said growth factor.

The invention also contemplates a method for the treatment of a DFU in a patient without criteria for amputation of the affected limb, at the time of the evaluation, that comprise administering to the patient a therapeutically effective amount of EGF, by intralesional infiltration in the ulcer base, once a week. The method of the invention allows the patient to be treated with conventional therapy during the course of the same treatment.

In one embodiment of the method of the invention, the amount of EGF per dose administered to the patient is between 2 and 80 µg. In the method of the invention, the evaluation of the DFU can be performed at the beginning, either at the time of the initial diagnosis, or during the course of a treatment to avoid amputation of the limb affected by the DFU.

In a particular embodiment of the method of the invention, before receiving the intralesional infiltration of EGF in a once-a-week schedule, the patient is treated three times per week with EGF administered by the same route in the ulcer base. This combined treatment schedule, while being effective in patients, allows the reduction of the possibility that they suffer the most frequent adverse events of intralesional administration of rhEGF, such as pain and burning, tremors and chills, exposing the patient to lower total doses of rhEGF by this route.

As can be seen in the invention, surprisingly, similar granulation results were obtained with the new treatment scheme proposed, in comparison with the established administration schedule, of three times a week during the 8 weeks, in patients who received comparable amounts of rhEGF by dose.

### Examples

### Example 1. Intralesional administration of rhEGF in three regimes of treatment for DFU patients.

The characteristics of the patients with DFU involved in the study, and their lesions, are observed in Table 1. It can be seen that the mean age was above 60 years old, ranging between 24 and 87 years. There were no differences in the gender of the patients. Type 2 diabetes predominated. The evolution time of diabetes was over 16 years, and the lesions were mostly Grade 3 in the Wagner classification. The patients were randomly assigned to one of the three treatment regimens, according to the scheme and dose of the rhEGF administered. The area of the lesion was greater than 20 cm² in the three groups.

Patients in groups 1 and 2 received rhEGF, at a rate of 75 µg/dose or 25 µg/dose, respectively, three times a week, intralesionally, in a way that involved the edges and base of the lesion. To do so, the reconstitution and dilution of the vial containing the drug was performed with 5 mL of water for injection. The administrations were made until 100% granulation of lesion, the closure of the lesion by grafting, or when a maximum of 8 weeks of treatment was attained.

Patients belonging to group 3 received the drug (75 µg/dose) three times a week, during the first week, by intralesional route. From the second week onward, the treatment continued with a dose of 225 µg, once a week. The treatment period was 8 weeks, although it was shorter if the complete closure of the lesion occurred earlier or if the ulcer reached a size smaller than 1 cm².

**Table 1. Demographic data of patients under study.**

| **Variable** | | **Group 1: 75 µg *n=45*** | **Group 2: 25 µg *n=45*** | **Group 3: 75 µg/ 225 µg *n=25*** |
|---|---|---|---|---|
| Age (years) | Mean ± ^{a}SD | 61.5 ± 11.1 | 62.3 ± 13.7 | 63.0 ± 9.0 |
| | Median ± ^{b}QR | 63.0 ± 16.0 | 65.5 ± 17.0 | 64.0 ± 13.0 |
| | (Min; Max) | (40; 87) | (24; 79) | (43; 83) |
| Gender | Male | 19 (42.2%) | 24 (53.3%) | 15 (60.0%) |
| | Female | 26 (57.8%) | 21 (46.7%) | 10 (40.0%) |
| Type of Diabetes | 1 | 7(15.6%) | 8 (17.8%) | - |
| | 2 | 38 (84.4%) | 37 (82.2%) | 25 (100.0%) |
| Time of evolution of diabetes (years) | Mean ± SD | 17.4 ± 10.2 | 17.8 ± 10.1 | 16.0 ± 12.0 |
| | Median ± QR | 19.5 ± 12.0 | 15.0 ± 15.0 | 12.0 ± 17.0 |
| | (Min; Max) | (1; 40) | (0; 40) | (2; 42) |
| Wagner Classification | 3 | 32 (71.1%) | 31 (68.9%) | 25 (100.0%) |
| | 4 | 13 (28.9%) | 14 (31.1%) | - |
| Lesion Area (cm²) | Mean ± SD | 25.4 ± 19.5 | 26.4 ± 26.0 | 22.2 ± 5.4 |
| | Median ± QR | 27.2 ± 34.3 | 20.1 ± 24.4 | 20.8 ± 10.3 |
| | (Min; Max) | 1.5; 97.2 | 5.2; 90.7 | (14.1; 30.0) |

| | | | | |
|---|---|---|---|---|
| ^{a}SD: Standard Deviation; ^{b}QR: Interquartile Range | | | | |

In all the groups, rhEGF obtained and purified starting from recombinant yeasts was used. The formulated product was lyophilized in vials containing 25 µg or 75 µg of the active pharmaceutical ingredient. The composition also contained pharmaceutically acceptable excipients, such as stabilizers and pH regulators. Patients in all three groups continued receiving conventional therapy, which was performed according to the DFU treatment standards established by the National Angiology Group of Cuba. This consists of: offloading of the lesion, metabolic control of the underlying disease with insulin (which replaces oral hypoglycemic agents), washing of the lesion with saline solution and mild soap substances, debridement, application of sterile dressings with gauze bandage, and antimicrobials, depending on the type of infectious germ present in the lesion.

The area of the DFU and the percentage of granulation were measured by a commercial system created for these purposes (Visitrak^{™}, Smith & Nephew), which consists of grid transparent films and a device to measure the lesion by planimetry. This system has been previously validated (Sugama J, et al. J Clin Nurs 2007; 16 (7): 1265-1269). Using a permanent marker, the tracing of the contours of the lesion and the granulated regions, on the grid transparent film, was conducted. The results obtained after the administration of the drug, by intralesional route, in patients with a high risk of amputation of the affected limb, can be seen in Table 2.

**Table 2. Granulation and closure of lesion response and amputations after the intralesional treatment with rhEGF.**

| **Response** | | **Group I: 75 µg** | **Group 2: 25 µg** | **Group 3: 225 µg** |
|---|---|---|---|---|
| | | **n= 45** | **n= 45** | **n= 25** |
| **Granulation** | **Objective Response: CR* + PR* (%)** | 34 (75%) | 29 (64%) | 17 (68%) |
| | **Complete Response: CR (%)** | 36 (80%) | 31 (69%) | 19 (76%) |
| **Lesion Closure** | | 34 (75%) | 27 (60%) | 18 (72%) |
| **Amputations** | | 6 (13%) | 10(22%) | 3 (12%) |

| | | | | |
|---|---|---|---|---|
| **CR*:** Complete granulation response at 3 weeks (≥ 75% of the area of the lesion covered by useful granulation tissue). **PR*:** Partial granulation response at 3 weeks (>50% and <75% of the area of the lesion covered by useful granulation tissue). **CR:** Complete granulation response at the end of treatment (100% of the area of the lesion covered by useful granulation tissue). | | | | |

In terms of granulation, at the end of week 3, in those patients that received the 75 µg dose (three times per week) during the whole treatment, as well as in those that received an initial dose of 75 µg (three times a week) followed by a weekly dose of 225 µg, the results were superior. The values reached in this last group were surprising. At the same point of ulcer measurement, 64% of granulation was found in the group of patients treated with the 25 µg dose (three times per week). Likewise, important results, of more than 70% lesion closure, and reduction of the risk of amputation, below 15% in groups 1 and 3, were obtained; that is, the lesions mostly passed from the situation of amputation to the non-amputation situation.

### Example 2. Determination of rhEGF levels in the plasma of DFU patients treated by intralesional route.

A pharmacokinetic study was carried out, starting from blood samples taken from 31 patients. The design was directed to determine kinetic parameters after the intralesional administration of rhEGF in the DFU. The most important parameters evaluated were the 'Area under the Curve' (AUC) and the half-life time (t½) among others. Patients were treated three times a week with the drug containing rhEGF at the following levels: 25 µg/dose, 75 µg/dose and 225 µg/dose. The treatment was performed for 8 weeks, or less time if the ulcer area reached a measure equal or less than 1 cm².

The demographic characteristics of the patients can be seen in Table 3. Men were the 56.2% of the patients. The average age was 63 years. Patients suffering Type 2 Diabetes were 81.2 %, with a wide range of variability in the time of evolution of diabetes, which oscillated between 8 and 41 years.

**Table 3. Demographic characteristics of patients involved in the study.**

| **Variable** | | **Group 1: 75 µg *n*=*8*** | **Group 2: 25 µg *n*=*8*** | **Group 3: 225 µg *n=25*** |
|---|---|---|---|---|
| Age (years) | Mean ± ^{a}SD | 55.3 ± 10.1 | 51.6 ± 11.4 | 63.0 ± 9.0 |
| | Median ± ^{b}QR | 58.0 ± 4.2 | 49.5 ± 17.4 | 64.0 ± 13.0 |
| | (Min; Max) | (39; 68) | (30; 59) | (43; 83) |
| Gender | Male | 5 (62.5%) | 4 (50.0%) | 15 (60.0%) |
| | Female | 3 (37.5%) | 4 (50.0%) | 10 (40.0%) |
| Diabetes Type | 1 | 2 (25.0%) | 1 (12.5%) | - |
| | 2 | 6 (75.0%) | 7 (87.5%) | 25(100%) |
| Time of evolution of diabetes (years) | Mean ± SD | 14.5 ± 22.7 | 23.0 ± 10.0 | 16.0 ± 12.0 |
| | Median ± QR | 18.0 ± 15.0 | 19.5 ± 14.3 | 12.0 ± 17.0 |
| | (Min; Max) | (8; 32) | (9; 41) | (2; 42) |
| Wagner | 1 | - | 1 (12.5%) | 25(100%) |
| Classification | 2 | 8 (100%) | 7 (87.5%) | - |
| Lesion Area (cm²) | Mean ± SD | 10.13 ± 12.42 | 9.14 ± 3.5 | 22.2 ± 5.4 |
| | Median ± QR | 4.11 ± 21.14 | 10.15 ± 9.33 | 20.8 ± 10.3 |

| | | | | |
|---|---|---|---|---|
| ^{a}SD: Standard Deviation; ^{b}QR: interquartile range | | | | |

The measurements of the protein (rhEGF), that is the active substance of the drug, were done just before the first administration (to), immediately after the first administration (t₁), and at different times after the first administration. The rhEGF concentration was determined in the plasma samples of the patients, by a commercially available ELISA kit (Quantikine^{®}, R&D), with a sensitivity <0.7 pg / ml. For the quantitation of this growth factor, the instructions of the kit manufacturer were followed.

Two hours of sample collection were enough to characterize the pharmacokinetic profile. Once this time was over, the rhEGF value returned to baseline levels, independently of the dose received, with no subsequent increases.

It can be observed in Table 4 that higher values of AUC were detected in the doses of 75 µg and 225 µg, if compared to those achieved with the dose of 25 µg. Patients who received this inferior dose had lower values of t_{1/2} respect to the doses of 75 µg and 225 µg.

**Table 4. Pharmacokinetic parameters estimated for different doses of rhEGF administered to patients with DFU.**

| **Studied Dose** | **Parameters** | Immediately after the first **administration** |
|---|---|---|
| **25 µg** | AUC (pg.h.ml⁻¹) | 85.1 ± 50.2 |
| | t_{1/2} (h) | 0.85 ± 0.78 |
| **75 µg** | AUC (pg.h.ml⁻¹) | 197.8 ± 73.9 |
| | t_{1/2} (h) | 0.66 ± 0.18 |
| **225 µg** | AUC (pg.h.ml⁻¹) | 288.6 ± 89.4 |
| | t_{1/2} (h) | 0.75 ± 0.21 |

From the bioavailability analysis performed, it was observed that, when doses of 25 µg and 75 µg are applied, a small fraction of protein passes into the blood. In the area of the ulcer remain about 19 µg and 58 µg, respectively. On the other hand, for the 225 µg dose, the greatest amount of rhEGF passes into the blood immediately, and only a small fraction remain in the area of the ulcer. This fraction is around 63 µg. Hence, an increase in the dose of the administered growth factor does not cause a greater concentration in the tissue. These results are in agreement with the fact that an increase in the dose of EGF does not lead to a greater effect.

### Example 3. Healing of DFU after the intralesional administration of rhEGF at different doses and administration schedules.

Taking into account the results obtained in Examples 1 and 2, a study was designed with patients of DFU, with amputation criteria of the affected limb, assaying different doses of rhEGF and an administration schedule where the frequency of a weekly administration of rhEGF, when the patient stop having amputation criteria of the limb, was included.

On one side, 48 patients were included, who received the treatment with rhEGF three times a week until it was confirmed that they no longer had an amputation criterion for the affected limb. This period of repeated applications in one week generally coincided with the first three weeks of therapy with injectable rhEGF. Then, the treatment with rhEGF was continued once a week. The route of administration was always the intralesional one. The complete treatment was carried out for 8 weeks, or for less time, if before the 8 weeks the ulcer base was already covered with 100% useful granulation tissue, or if the lesion already reached an area ≤ 1 cm². The doses administered per group were 2.5 µg, 25 µg, 50 µg and 75 µg. Seven patients were incorporated into the group that received 2.5 µg, 10 patients to the group that received the 25 µg dose; 13 patients to the group that received 50 µg and 18 patients in the group of 75 µg.

On the other hand, there was a group of 93 patients who were treated, during the whole period, with the dose of 75 µg three times a week. The period of treatment was 8 weeks. At the end of the study, the results of this group of patients were used as a control, in comparison with the rest of the groups that received the treatment schedule described in the previous paragraph.

Among patients, the masculine gender predominated. The average age was 64 years, Type 2 Diabetes prevailed. The ulcer dimension was between 22 and 27 cm², and in the majority of patients the lesions were classified as Grade 3 and 4 of the Wagner classification. Patients in all groups continued receiving the conventional therapy, which was performed according to the DFU standard treatment established by the National Angiology Group of Cuba, as summarized in Example 1.

The results obtained are shown in Table 5. It is important to note that similar granulation results were obtained with the new proposed treatment schedule, in comparison with the established schedule of administration, of three times a week during 8 weeks, in those patients that received the same amount of rhEGF per dose.

**Table 5. Results of the intralesional administration of rhEGF in patients treated with different doses and administration schedules.**

| **Response to treatment** | | **Treatment Group** | | | | **Control Group** |
|---|---|---|---|---|---|---|
| | | **2.5 µg** | **25 µg** | **50 µg** | **75 µg** | **75 µg** |
| | | ***n*=*7*** | ***n*= *10*** | ***n*= *13*** | ***n= 18*** | ***n=93*** |
| Granulation | Objective | 4 (57%) | 6 (60%) | 8 (61%) | 13 (72%) | 72 (77%) |
| | Complete | 5 (71%) | 7 (70%) | 10 (77%) | 15 (83%) | 79 (85%) |

| | | | | | | |
|---|---|---|---|---|---|---|
| *Objective granulation response:* ≥ 75% of the lesion area covered by useful granulation tissue, at 3 weeks. *Complete granulation response*: 100% of the lesion area covered by useful granulation tissue, at 8 weeks. | | | | | | |

During the follow-up, in the groups that received the combined schedule of three administrations per week, followed by one administration per week, the closure of the lesion was obtained in 4 patients of the 2.5 µg group (57%), 5 patients in the 25 µg group (50%), 9 patients in the 50 µg group (69%) and 13 patients in the 75 µg group (72%). In the treatment group that served as a control, where patients received three administrations of rhEGF per week (75 µg), the closure of the lesion was observed in 81% of the patients. Likely, amputations were below 12% in the four groups that differed in the rhEGF dose, and in the group that served as a control. Surprisingly, the new treatment schedule proposed here, along with conventional therapy, led to a reduction in the risk of amputation of more than 80%.

This combined treatment schedule, while being effective in patients, allows reducing the possibility that they suffer the most noticeable adverse events of intralesional administration of rhEGF, such as pain and burning, tremors and chills, by exposing the patient in fewer occasions to the administration of rhEGF given by this route.

### Example 4. Healing of DFU after intralesional administration of rhEGF once a week.

A series of cases was treated with conventional therapy, like the one described in Example 1, plus rhEGF at a dose of 2.5 µg (10 patients), 25 µg (15 patients), 50 µg (15 patients) or 75 µg (20 patients). The intralesional treatment with this growth factor was performed once a week, for 8 weeks, or for less time, if the lesion closed up to a dimension equal to or less than 1 cm². Another group of patients received conventional therapy only, with the difference that the procedure was performed three times a week, until the complete closure of the lesion was achieved.

The patients in these series had no amputation criteria of the limb affected by the DFU at the time of the initial evaluation. In general, the higher percentage of patients was female for all groups, both in those prescribed with rhEGF and in those that this medication was not indicated. Type 2 diabetes prevailed, with a median evolution of 15 years. The ulcer size ranged from 2 cm² to more than 20 cm². As mentioned before, conventional therapy in all patients was performed according to the standards of treatment for DFU established by the National Angiology Group of Cuba.

Table 6 shows the results, in terms of lesion closure between 8 and 12 weeks after commencement of treatment. When the therapeutic procedure that includes intralesional rhEGF, administered once a week, is compared with conventional therapy only, surprisingly, much better results are observed, in favor of the administration of rhEGF in the four groups of patients. The lesion closure in all groups treated with rhEGF was between 60% and 80%, while in the group that received only conventional therapy the lesion closure was observed in 45% of the individuals.

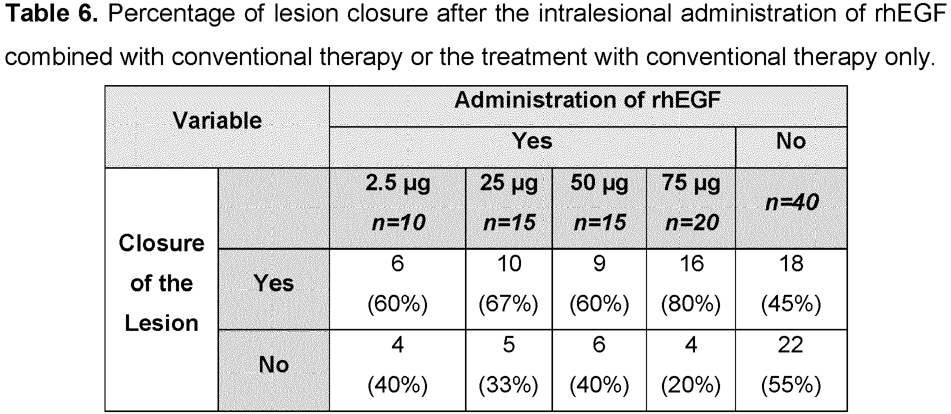

### Example 5. Effect of injectable rhEGF administered once a week in patients with DFU with a diagnosis of non-amputation of the affected lower limb.

To confirm the effect of the intralesional administration of injectable rhEGF, given once a week, had on the healing of DFU, patients who had no criteria for amputation of the affected lower limb at the time of the initial evaluation or diagnosis were treated.

*Patient A*: Male diabetic patient, 62 year old, with 15 years of evolution of diabetes, who presented a DFU with the following characteristics: superficial lesion that does not reach the deep skin planes and without signs of infection, that at the physical examination, and by hemodynamic parameters and Transcutaneous Oxygen Pressure (TcPO₂), did not have moderate or critical ischemia. The size of the lesion, after debridement, was 10 cm². The patient received rhEGF, at a dose of 75 µg, once a week, for two weeks, and after said time the complete closure of the lesion was attained.

*Patient B*: Male diabetic patient, 54 years old, with 10 years of evolution of diabetes, who presented a DFU with the following characteristics: deep lesion that involved joint capsule and ligaments, but without infection or associated osteomyelitis, that at the physical examination, and by hemodynamic parameters and TcPO₂, did not have moderate or critical ischemia. The ulcer base was covered with more than 75% of useful granulation tissue. The ulcer size was 7 cm². The patient received rhEGF, at a dose of 75 µg, once a week, for two weeks. A complete granulation response was obtained after said time, and closure of the lesion at 9 weeks after the first application was achieved.

*Patient C*: Female diabetic patient, 68 years old, with 18 years of evolution of diabetes, who has a DFU with the following characteristics: deep lesion that involved joint capsule and ligaments, but without infection and associated osteomyelitis, that at physical examination, and by hemodynamic parameters and TcPO2, did not have moderate or critical ischemia. The ulcer size was 7 cm². This patient did not receive rhEGF, but she was treated with the conventional therapy. The patient was kept in a follow up evaluation. In the absence of said drug, a complete granulation response was obtained at 14 weeks, and at 20 weeks the DFU closure was not obtained yet.

## Claims

1. Use of Epidermal Growth Factor (EGF) for the manufacture of an injectable medicament for the treatment of a Diabetic Foot Ulcer (DFU) in a patient without criteria of amputation of the affected limb at the time of evaluation, wherein the medicament comprising EGF is administered by intralesional infiltration into the ulcer base once a week.

2. The use of claim 1 wherein the amount of EGF per dose of the medicament administered to the patient is between 2 and 80 µg.

3. The use of claim 1 wherein the evaluation of the DFU is performed at the beginning or during the course of a treatment.

4. The use of claim 1 wherein the DFU was treated three times per week with EGF administered intralesionally before the treatment by intralesional infiltration with EGF in the base of said ulcer once a week.

5. The use of claim 1 wherein the patient is treated with conventional therapy during the course of the same treatment.

6. A pharmaceutical composition for the treatment of a Diabetic Foot Ulcer (DFU) in a patient without criteria of amputation of the affected limb at the time of evaluation comprising Epidermal Growth Factor (EGF) and pharmaceutically acceptable excipients, wherein the composition is to be administered once a week.

7. The composition of claim 6 wherein the amount of EGF is between 2 and 80 µg per dose that is administered to the patient.

8. Method of treatment of a Diabetic Foot Ulcer (DFU) in a patient without criteria of amputation of the affected limb at the time of evaluation comprising administering to the patient a therapeutically effective amount of Epidermal Growth Factor (EGF) by intralesional infiltration into the ulcer base once a week.

9. The method of claim 8 wherein the amount of EGF per dose administered to the patient is between 2 and 80 µg.

10. The method of claim 8 wherein the evaluation of the DFU is performed at the beginning or during the course of a treatment.

11. The method of claim 8 wherein the DFU was treated three times per week with EGF administered intralesionally before the treatment by intralesional infiltration with EGF in the ulcer base once a week.

12. The method of claim 8 wherein the patient is treated with conventional therapy during the course of the same treatment.
